# EUROPEAN PATENT APPLICATION

(11) **EP 3 151 193 A1**
(43) Date of publication of application: **05.04.2017**
(21) Application number: 16190773.8
(22) Date of filing: 27.09.2016
(51) Int. Cl.: G06T 7/00, G06T 15/00

(54) **SYSTEM FOR ASSESSING BONE FUSION**

(30) Priority: 30.09.2015 US 201562235038 P; 20.09.2016 US 201615270852
(71) Applicant: Curvebeam LLC, Warrington, PA 18976 (US)
(72) Inventor: MUNDRY, Uwe Hans, Landrum, SC 29356 (US); SINGH, Arun, North Wales, PA 19454 (US)
(74) Representative: Earnshaw, Geoffrey Mark

(57) **Abstract**

A system is disclosed for assessing or depicting the degree of bone fusion in an area. The system involves receiving three dimensional image data from a patient of a portion of a patient's body. Using the data, the scanned portion of the patient's body is reconstructed. The system permits a user to select a volume within the reconstructed volume which defines a volume of interest. The density of each voxel is detected within the volume of interest. An analysis is conducted on the voxel densities to determine the amount of fusion that has occurred, the higher the density in a prescribed area the greater the amount of fusion that has occurred. The system generates a depiction of the density.

## Description

### Field of the Invention

The present invention is directed to a method and system to assess the state or progress of bone fusion in the human body based upon digitally created 3D volumes.

### Background

Orthopedic surgeons often need to assess the state of bone fusion in a fracture or break in order to prescribe the next steps in a patients' therapy. Bone fusion is a natural healing process after an intentional or accidentally caused fracture, or when two separate bones are intentionally joined together through the use of fixation hardware (arthrodesis). Allowing a patient to bear weight on a fused bone is dictated by how well the bone portions have fused.

Current methods for assessing bone fusion are based upon analyzing an image of the area of interest using a digital filter edge detection process which seeks to detect density changes. The higher the degree of fusion that has occurred, the higher the density of the fused area, which results in a better visibly detectable edge amplitude. In order to provide this assessment, current methods typically use standard two dimensional X-ray radiographs upon which the edge detection is performed and qualitatively evaluated.

### Summary of the Invention

In one embodiment of the present invention the volume of interest that includes the location of the fusion in the patient is scanned using a three dimensional scanning technique, such as a computer tomography (CT) system.

An analysis of the volume of interest is conducted using a volumetric histogram of the voxel elements present within the volume of interest. A ratio of the number of high density voxels over all voxels is calculated. The ratio provides a value indicative of the state of fusion.

It is also contemplated that a series of assessments can be conducted over a period of time (e.g., days, weeks, etc.). The change in the ratio over a period of time allows for the monitoring of progress of the fusion process, and the rate of change in fusion, thus permitting more accurate assessment of healing.

In one embodiment, the system performs the steps of
receiving three dimensional image data of a portion of a patient's body;
reconstructing the scanned portion of the patient's body using the scanned image data;
permitting a selection of a volume within the reconstructed volume, the selection defining a volume of interest;
detecting density of each voxel within the volume of interest;
analyzing the detected densities of the voxels within the volume of interest to determine the amount of fusion that has occurred, the higher the density in a prescribed area the greater the amount of fusion that has occurred; and
determining a change in density over a prescribed time.

Optionally, the analyzing of the density of the voxels involves counting the total number of voxels for each density value, sorting the number of voxels for each density value, and plotting the number of voxels based on their density value, wherein the peaks of the plotted values at higher densities are indicators of bone fusion.

The system may include the optional steps of storing data representing the plotted number of voxels based on their density value, and/or retrieving previously stored data representing the plotted number of voxels based on their density value; where the step of plotting the number of voxels based on their density involves depicting graphs of the stored data and current data; and where the step of determining a change in density over a prescribed time involves analyzing the two graphs for determining at least one of increases in density or rate of change of density.

Optionally, the system calculates a percentage change and outputting the percentage.

Preferably the selected volume of interest is a location on the patient where a fracture/arthrodesis was present and also preferably the adjacent bone structure. Preferably the step of analyzing the density of the voxels involves analyzing voxels in both the area where the fracture/arthrodesis was located and the adjacent bone structure; and the system provides a comparison of the analyzed density data for both areas.

A computer program is also disclosed for assessing bone fusion. The program includes a reconstruction module that is configured to receive three dimensional image data of a portion of a patient's body and to depict on a computer monitor a reconstructed volume of the scanned portion of the patient's body using the scanned image data. A selection module is configured to allow a user to select a volume within the reconstructed volume, the selection defining a volume of interest. The program includes an analyzing module which detects the density of each voxel within the volume of interest and analyzes the detected densities of the voxels within the volume of interest to determine an amount of density associated with each voxel within the volume of interest. The analyzing module calculates an amount of bone fusion that has occurred base on the analysis of the detected densities.

The program outputs onto the monitor a generated depiction of the density variations across the volume of interest.

Also disclosed is a computer program product comprising machine readable instructions residing on storage means, which when loaded and executed on a microprocessor of a system, cause the system to:
receive three dimensional image data of a portion of a patient's body;
reconstruct the scanned portion of the patient's body using the scanned image data;
permit a selection of a volume within the reconstructed volume, the selection defining a volume of interest;
detect density of each voxel within the volume of interest;
analyze the detected densities of the voxels within the volume of interest to determine the amount of fusion that has occurred, the higher the density in a prescribed area the greater the amount of fusion that has occurred; and
generate a depiction of the density variations across the volume of interest.

The foregoing and other features of the invention and advantages of the present invention will become more apparent in light of the following detailed description of the preferred embodiments, as illustrated in the accompanying figures. As will be realized, the invention is capable of modifications in various respects, all without departing from the invention. Accordingly, the drawings and the description are to be regarded as illustrative in nature, and not as restrictive.

### Brief Description of the Drawings

For the purpose of illustrating the invention, the drawings show a form of the invention which is presently preferred. However, it should be understood that this invention is not limited to the precise arrangements and instrumentalities shown in the drawings.
Fig. 1 illustrates an imaging process of a patient's foot that includes a volume of interest.
Fig. 2 illustrates a computer generated reconstruction of the patient's foot using the scanned data from the process in Fig. 1.
Fig. 3 illustrates a volume of interest defined around the area of interest in the reconstructed volume of Fig. 2.
Fig. 4 is a depiction of a volumetric histogram created from density analysis of the volume of interest in Fig. 3.

### Detailed Description of the Embodiments

The state or progression of bone fusion in the human body often needs to be known for a variety of reasons. In particular, a patient typically is only allowed to bear weight on an injured bone, or post-arthrodesis, once the fusion is strong enough to allow for such weight bearing. If the fused area is stressed too early on in the fusion process, breakage (or rebreakage) can occur leading to a variety of clinical complications.

A three dimensional scanning system, such as a computed tomography (CT) system is utilized to capture or acquire the entire volume around a fracture/arthrodesis location of a bone. This is typically done by rotating a data acquisition system around a patient, thereby creating an accurate, undistorted three dimensional dataset of the scanned volume. Preferably the system uses a high resolution CT scanner capable of sub-millimeter voxels.

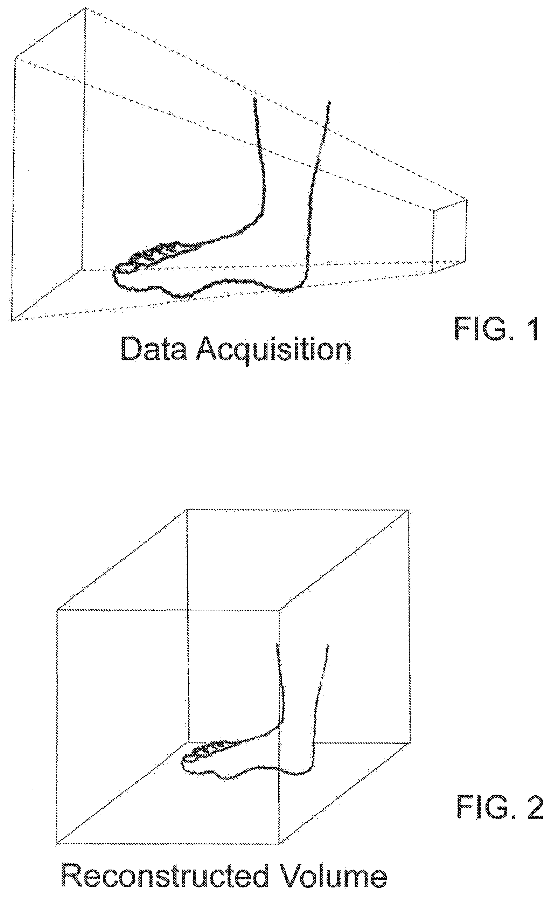

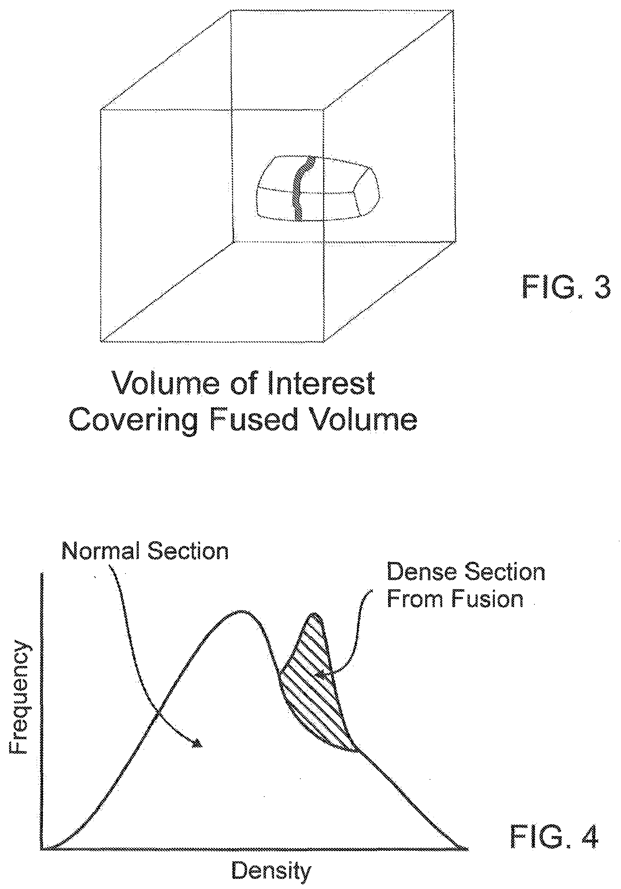

The system reconstructs a three dimensional volume of the scanned portion of the patient. Fig. 2 The user (doctor or technician) uses a feature of the system that allows the defining of a volume of interest surrounding a portion, or sub volume of the entire 3D volume. Fig. 3. The volume of interest includes the area where the fusion is occurring and the adjacent bone. Using a conventional density analysis program, density representations of thousands or millions of tiny volume elements (also known as voxels) are detected. Once all the voxels within that volume of interest are detected, they are then analyzed to define the degree of fusion.

More specifically, the voxel analysis is conducted by counting voxels based on their individual density values, sorting the counts by voxel value and plotting their frequencies (binning the densities). With this data, the system creates a volumetric histogram. Fig. 4. The volume histogram will show more or less sharp peaks at the higher end of the voxel density range and the peaks will be a measure of the amount of fusion achieved.

Characterizing such peaks leads to a measure of the state of the fusion process. The higher the percentage of voxel counts at higher voxel densities, the more the fusion process will have progressed.

In one embodiment of the invention, several fusion assessments for a patient are determined over a period of time, such as daily, weekly, monthly, etc. The assessments permit the degree of bone fusion to be comparatively analyzed so as to provide information on the progress of the healing. The system can calculate the percentage increase in fusion and allow it to be plotted over time to give the doctor or technician the ability to see whether the bone fusion is increasing more or less quickly.

A database can be created and measurements can be compared to a collection of previously evaluated and stored values increasing the accuracy of the present fusion assessment method.

It is contemplated that the fusion assessment method described above will be part of an imaging software system which would allow for an accurate 3-dimensional placement of the volume of interest. Information about the location and dimensions of the volume of interest used can be recorded on a per-patient basis, to allow for the longitudinal assessment of the healing process over time, using identical volume of interest metrics.

The system or systems described herein may be implemented on any form of microprocessor. The system of the present invention may include a software program stored on the microprocessor and/or storage device (e.g., media). The method may be implemented through program code or program modules stored on a non-volatile computer-readable storage medium.

For the purposes of promoting an understanding of the principles of the invention, reference has been made to the preferred embodiments illustrated in the drawings, and specific language has been used to describe these embodiments. However, no limitation of the scope of the invention is intended by this specific language, and the invention should be construed to encompass all embodiments that would normally occur to one of ordinary skill in the art.

The particular implementations shown and described herein are illustrative examples of the invention and are not intended to otherwise limit the scope of the invention in any way. For the sake of brevity, conventional electronics, control systems, software development and other functional aspects of the systems (and components of the individual operating components of the systems) may not be described in detail.

Finally, the use of any and all examples, or exemplary language (e.g., "such as") provided herein, is intended merely to better illuminate the invention and does not pose a limitation on the scope of the invention unless otherwise claimed. Numerous modifications and adaptations will be readily apparent to those skilled in this art without departing from the spirit and scope of the invention.

## Claims

1. A system for assessing bone fusion, the system comprising the steps of:
receiving three dimensional image data of a portion of a patient's body;
reconstructing the scanned portion of the patient's body using the scanned image data;
permitting a selection of a volume within the reconstructed volume, the selection defining a volume of interest;
detecting density of each voxel within the volume of interest;
analyzing the detected densities of the voxels within the volume of interest to determine the amount of fusion that has occurred, the higher the density in a prescribed area the greater the amount of fusion that has occurred; and
generating a depiction of the density variations across the volume of interest.

2. A system for assessing bone fusion according to claim 1, wherein the analyzing of the density of the voxels involves counting the total number of voxels for reach density value, sorting the number of voxels for each density value, and plotting the number of voxels based on their density value, wherein the peaks of the plotted values are indicators of bone fusion.

3. A system for assessing bone fusion according to claim 1 or claim 2, further comprising the step of storing data representing the plotted number of voxels based on their density value.

4. A system for assessing bone fusion according to any preceding claim, further comprising the step of retrieving previously stored data representing the plotted number of voxels based on their density value; wherein the step of plotting the number of voxels based on their density involves depicting graphs of the stored data and current data; and wherein the step of determining a change in density over a prescribed time involves analyzing the two graphs for determining at least one of increases in density or rate of change of density.

5. A system for assessing bone fusion according to claim 4, further comprising the step of calculating a percentage change and outputting the percentage.

6. A system for assessing bone fusion according to any preceding claim, wherein the selected area of interest is a location on the patient where a fracture/arthrodesis was present.

7. A system for assessing bone fusion according to any preceding claim, wherein the three dimensional image data is captured using a high resolution CT scanner capable of generating sub-millimeter voxels.

8. A system for assessing bone fusion according to any preceding claim, wherein the volume of interest includes the area where the fracture/arthrodesis was located and the adjacent bone structure.

9. A system for assessing bone fusion according to claim 8, wherein the step of analyzing the density of the voxels involves analysing voxels in both the area where the fracture/arthrodesis was located and the adjacent bone structure; and wherein the system provides a comparison of the analysed density data for both areas.

10. A system for assessing bone fusion according to claim 9, further comprising the step of retrieving previously stored data representing the plotted number of voxels based on their density value; wherein the step of plotting the number of voxels based on their density involves depicting graphs of the stored data and current data; and wherein the step of determining a change in density over a prescribed time involves analyzing the two graphs for determining at least one of increases in density or rate of change of density.

11. A computer program product comprising machine readable instructions residing on storage means, which when loaded and executed on a microprocessor of a system, cause the system to:
receive three dimensional image data of a portion of a patient's body;
reconstruct the scanned portion of the patient's body using the scanned image data;
permit a selection of a volume within the reconstructed volume, the selection defining a volume of interest;
detect density of each voxel within the volume of interest;
analyze the detected densities of the voxels within the volume of interest to determine the amount of fusion that has occurred, the higher the density in a prescribed area the greater the amount of fusion that has occurred; and
generate a depiction of the density variations across the volume of interest.
